# EUROPEAN PATENT APPLICATION

(11) **EP 2 589 368 A1**
(43) Date of publication of application: **08.05.2013**
(21) Application number: 11800780.6
(22) Date of filing: 27.06.2011
(51) Int. Cl.: A61J 3/00, A61M 39/02

(54) **CONNECTOR AND CONNECTOR ASSEMBLY**

(30) Priority: 30.06.2010 JP 2010150077
(71) Applicant: Terumo Kabushiki Kaisha, Tokyo 151-0072 (JP)
(72) Inventor: TAKEMOTO, Masafumi, Fujinomiya-shi Shizuoka 418-0004 (JP)
(74) Representative: TBK
(86) International application number: PCT/JP2011/064659
(87) International publication number: WO 2012/002316

(57) **Abstract**

A first connector (2) includes a first connector main body configured from an outer tube (4) of a cylindrical shape, an inner tube of a cylindrical shape, and a hub (12) disposed at a proximal end portion of the outer tube (4), a hollow needle supported on the hub (12), a first sealing member supported on the inner tube, a coil spring serving as puncturing portion biasing means for biasing the first sealing member toward a distal direction, and a clamping member disposed upon the outer tube (4). Upon an inner periphery portion of the proximal end portion of the outer tube (4), a pawl (44) is formed so as to project to the inner side. In addition, upon an outer periphery portion of a large diameter portion (1212) of a main body portion (121) of the hub (12), a plurality of ratchet teeth (126) which are engageable with the pawl (44) of the outer tube (4) are formed so as to project toward the outer side. Accordingly, the rotation in only one of the forward and backward directions of the hub (12) is prevented.

## Description

### Technical Field

The present invention relates to a connector and a connector assembly.

### Background Art

Usually, a drug such as an anti-cancer agent or an immunosuppressive agent which is dangerous if a health care worker touches by mistake is accommodated in a state of powder in a vial container which is sealed at a mouth portion thereof with a rubber stopper.

In order to take out the drug from such a vial container as just described, the following operations are carried out.
First, the mouth portion of the vial container and a mouth portion of a syringe into which liquid for dissolving has been dealt out are connected to each other through a connector assembly configured from a first connector and a second connector. In this instance, a lock adapter having screw threads formed on an inner circumferential face thereof is provided on an outer circumferential portion of the mouth portion of the syringe (refer to, for example, Patent Document 1), and when the syringe is to be connected to a hub of the first connector, the lock adapter of the syringe is screwed on a screw threaded portion formed on the hub of the first connector. Consequently, the hub of the first connector and the mouth portion of the syringe are connected to each other, and the syringe is held on the hub of the first connector. Then, the first connector and the second connector which is connected to the mouth portion of a vial container are connected to each other.

Then, liquid for dissolving is injected into the vial container from the syringe through the connector assembly. Then, a pumping operation is carried out or the vial container is shaken to dissolve the drug uniformly into the liquid for dissolving. Thereafter, the liquid for dissolving (hereinafter referred to as "solution") in which the drug is dissolved is sucked out and taken out into the syringe.

However, in the conventional connector assembly described above, since the syringe is held on the hub of the first connector by the lock adapter, even if the syringe is pulled, it is not separated. However, the conventional connector assembly has a drawback that, if the syringe or the lock adapter is rotated in a direction in which the screwing engagement is loosened, then it is separated simply. If the syringe is separated from the hub of the first connector, then the solution may scatter from the mouth portion of the syringe or the like and may stick to the health care worker or the like. Therefore, there is the possibility that the solution may not be fed with safely and certainty through the connector assembly.

Patent Document 1: Japanese Patent No. 3456241

### Disclosure of Invention

The object of the present invention contemplates provision of a connector and a connector assembly wherein inadvertent separation of a medical device can be prevented.
In order to achieve the object described above, the present invention provides a connector, including:
an outer tube;
a hub disposed at a proximal end portion of the outer tube for rotation around an axis of the outer tube with respect to the outer tube, wherein the hub includes a main body portion inserted in the outer tube, a connecting portion having a proximal end side to which a medical device having a screw threaded portion is to be connected, and a projecting portion for being screwed with the screw threaded portion;
separation preventing means for preventing separation of the hub from the outer tube; and
rotation blocking means having a rotation blocking function for blocking the rotation of the hub in only one of a forward direction and a backward direction;
wherein the rotation blocking means includes a first engaging portion provided at the proximal end portion of the outer tube and a second engaging portion provided on the hub and engageable with the first engaging portion,
wherein a direction in which the hub is urged to rotate by rotational torque applied to the hub when the medical device is rotated with respect to the hub to screw the screw threaded portion with the projecting portion and a direction of the rotation of the hub blocked by the rotation blocking means coincide with each other, and
wherein the rotation blocking function of the rotation blocking means is rendered effective when the first engaging portion and the second engaging portion are engaged with each other.

In the connector of the present invention, preferably, one of the first engaging portion and the second engaging portion is a pawl and the other of the first engaging portion and the second engaging portion is a ratchet tooth engageable with the pawl.

In the connector of the present invention, preferably, the ratchet tooth includes a plurality of ratchet teeth, and the plurality of ratchet teeth are provided along a circumferential direction.

In the connector of the present invention, preferably the medical device includes a syringe having a mouth portion at a distal end portion thereof; and
in the connector, the mouth portion of the syringe is connected to the connecting portion.

Further, in order to achieve the object described above, the present invention provides a connector assembly, including:
the connector according to the present invention; and
a partner connector connected to the connector and having a connecting portion, wherein the connecting portion connects, at a distal end side of the connecting portion, a liquid accommodating container capable of accommodating liquid.

In the connector of the present invention, preferably the entire hub is inserted in the outer tube.
In the connector of the present invention, preferably the projecting portion is screw threads or a protrusion in the form of a flange.

In the connector of the present invention, preferably a lock adapter having the screw threaded portion on an inner circumferential face thereof is provided on an outer circumferential portion of the mouth portion of the syringe, and,
when the projecting portion and the screw threaded portion are screwed with each other, the hub portion is accommodated in the lock adapter.

### Brief Description of Drawings

[Fig. 1]

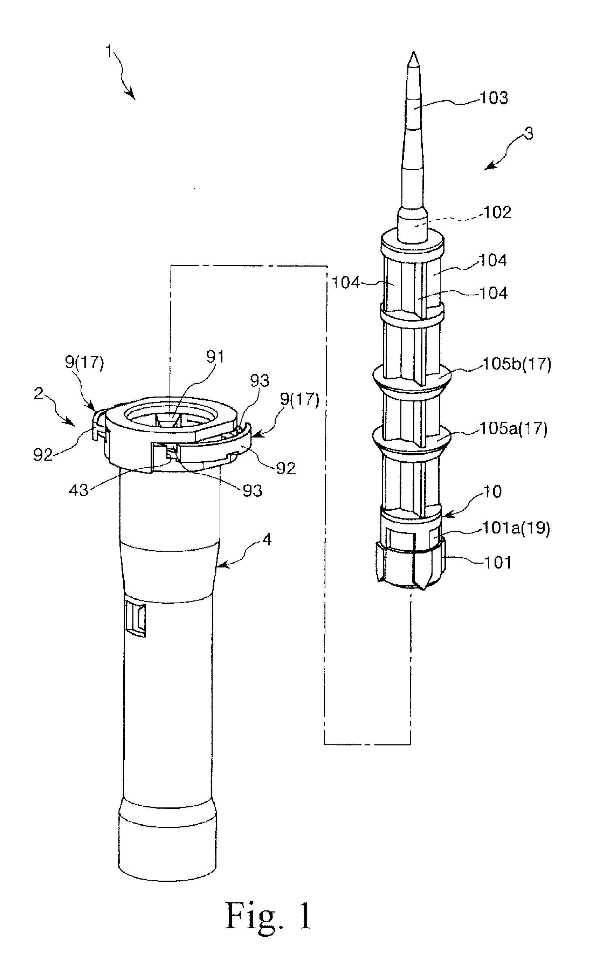

   Fig. 1 is an exploded perspective view showing an embodiment of a connector assembly of the present invention.
[Fig. 2]

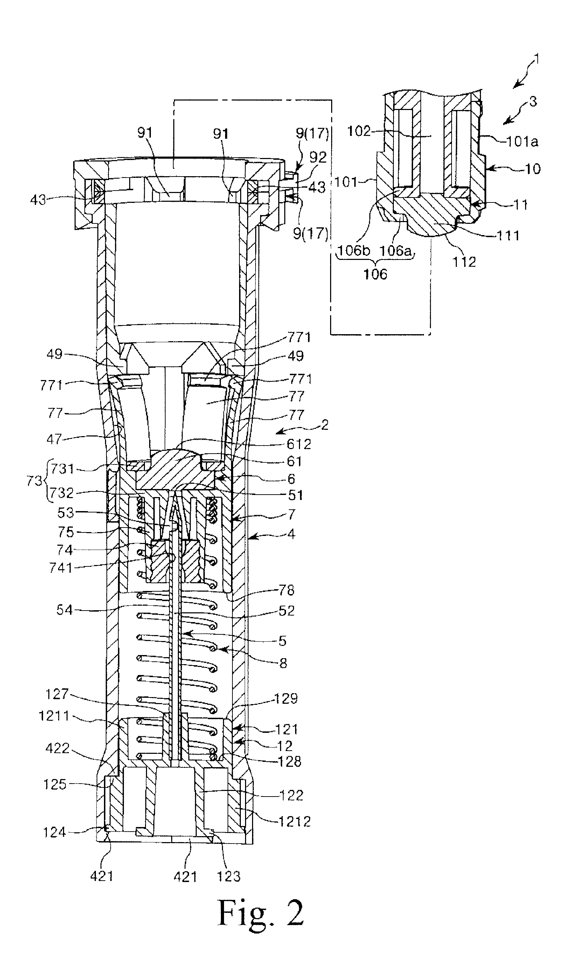

   Fig. 2 is a vertical sectional view illustrating a process until the first connector and the second connector of the connector assembly shown in Fig. 1 are placed into the assembled state.
[Fig. 3]

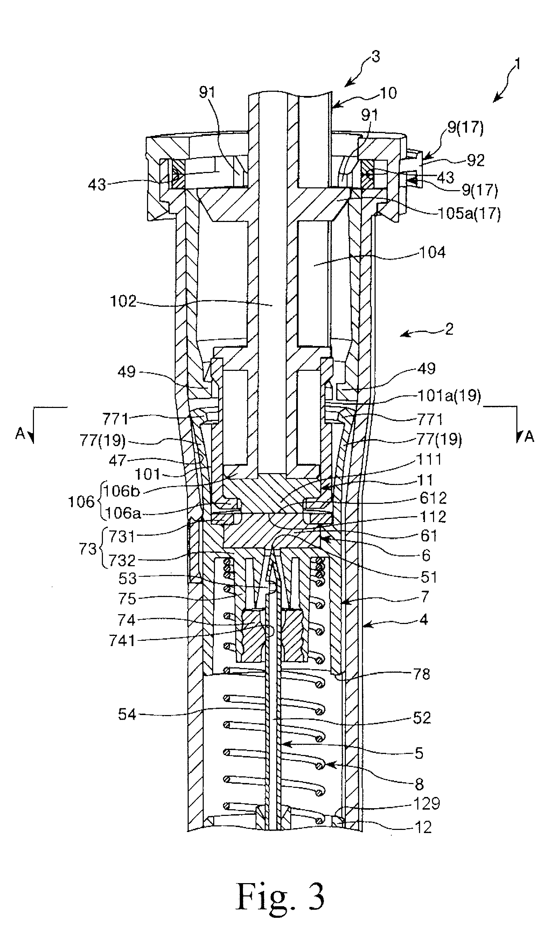

   Fig. 3 is a vertical sectional view illustrating a process until the first connector and the second connector of the connector assembly shown in Fig. 1 are placed into the assembled state.
[Fig. 4]

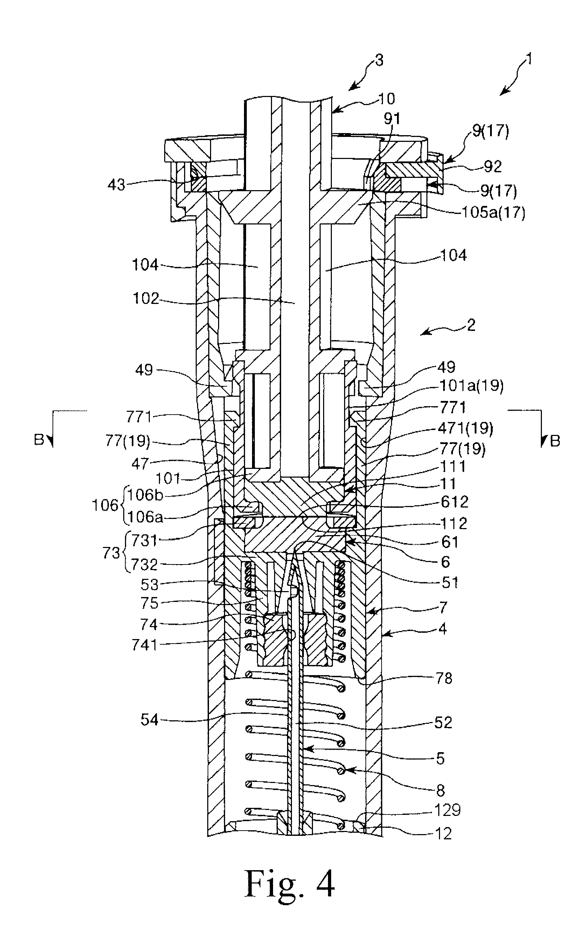

   Fig. 4 is a vertical sectional view illustrating a process until the first connector and the second connector of the connector assembly shown in Fig. 1 are placed into the assembled state.
[Fig. 5]

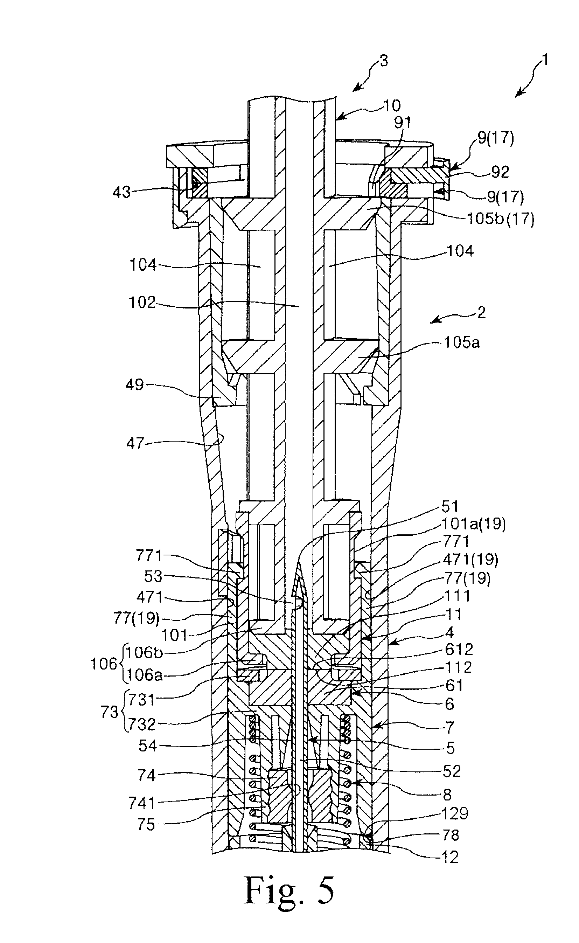

   Fig. 5 is a vertical sectional view illustrating a process until the first connector and the second connector of the connector assembly shown in Fig. 1 are placed into the assembled state.
[Fig. 6]

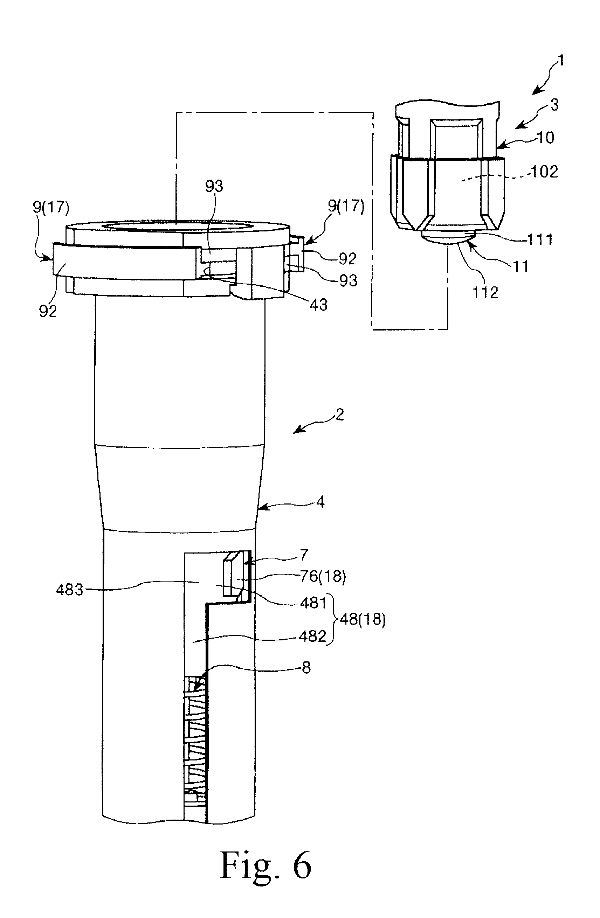

   Fig. 6 is a perspective view (view corresponding to Fig. 2) illustrating a process until the first connector and the second connector of the connector assembly shown in Fig. 1 are placed into the assembled state.
[Fig. 7]

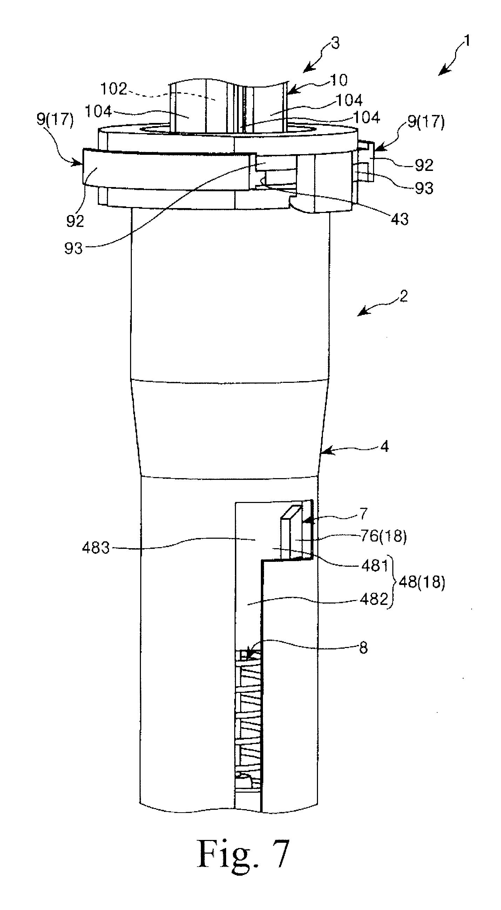

   Fig. 7 is a perspective view (view corresponding to Fig. 3) illustrating a process until the first connector and the second connector of the connector assembly shown in Fig. 1 are placed into the assembled state.
[Fig. 8]

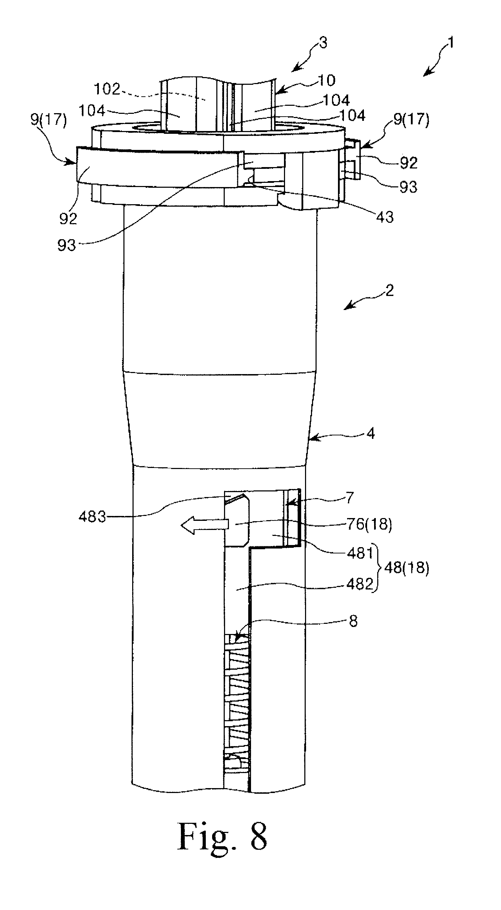

   Fig. 8 is a perspective view (view corresponding to Fig. 4) illustrating a process until the first connector and the second connector of the connector assembly shown in Fig. 1 are placed into the assembled state.
[Fig. 9]

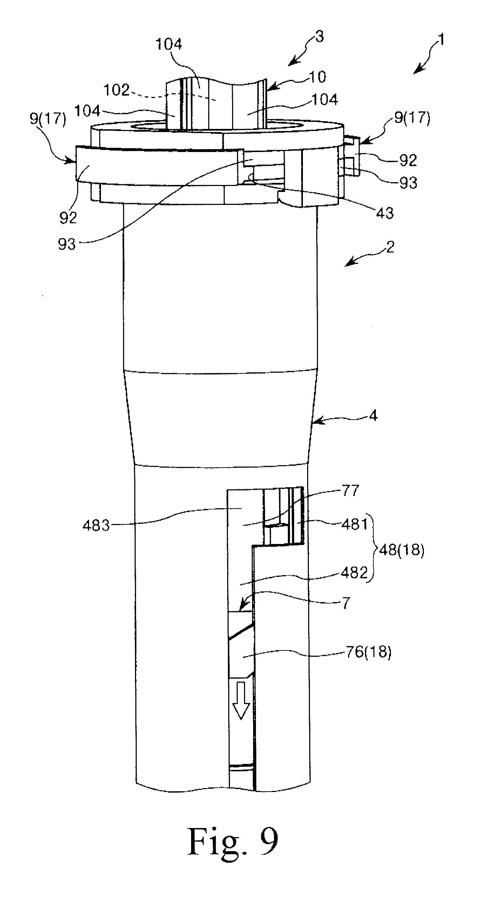

   Fig. 9 is a perspective view (view corresponding to Fig. 5) illustrating a process until the first connector and the second connector of the connector assembly shown in Fig. 1 are placed into the assembled state.
[Fig. 10]

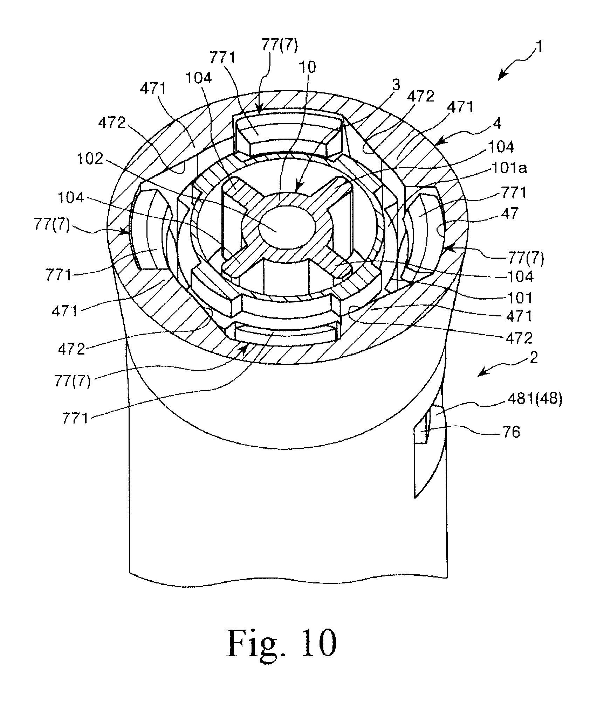

   Fig. 10 is a sectional view taken along line A-A of Fig. 3.
[Fig. 11]

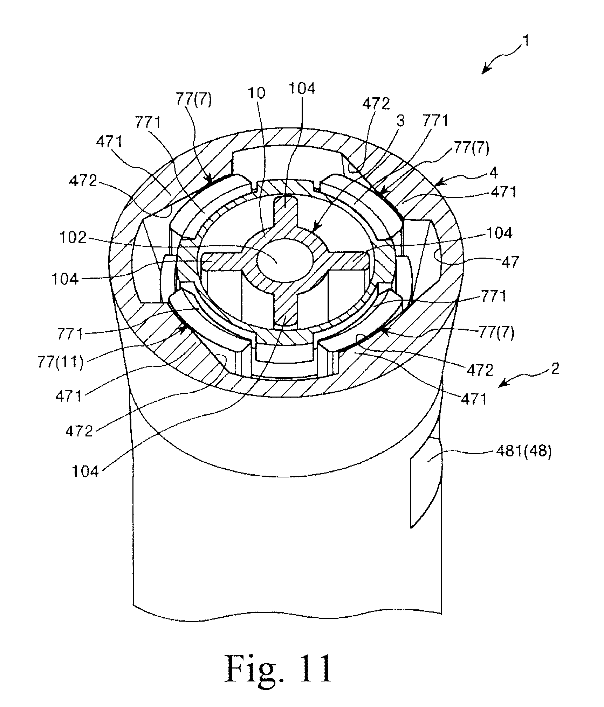

   Fig. 11 is a sectional view taken along line B-B of Fig. 4.
[Fig. 12]

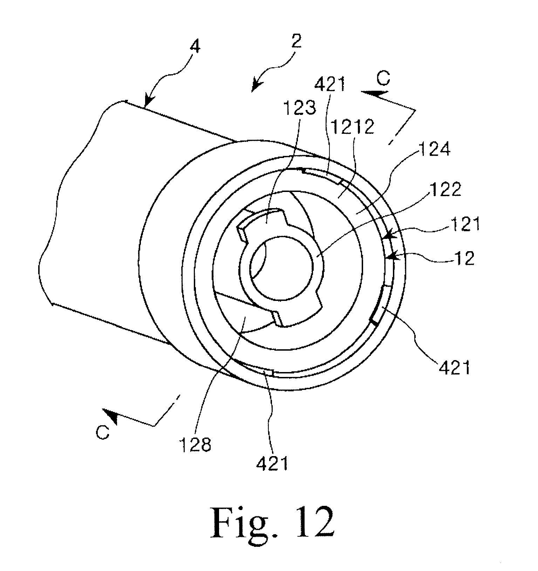

   Fig. 12 is a perspective view showing elements in the proximity of a proximal end portion of the first connector of the connector assembly shown in Fig. 2.
[Fig. 13]

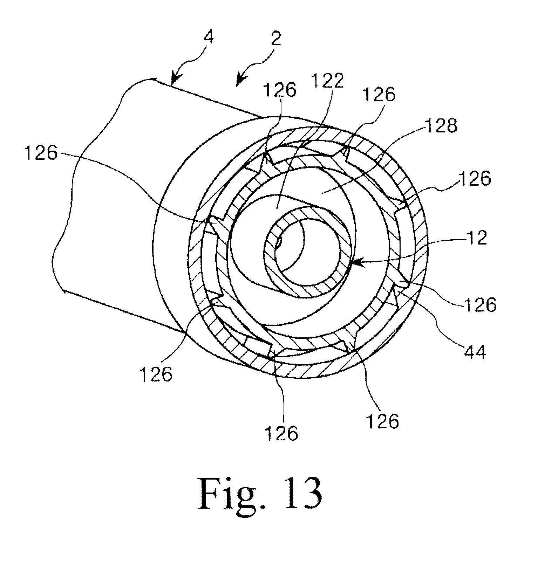

   Fig. 13 is a sectional perspective view taken along line C-C of Fig. 12.
[Fig. 14]

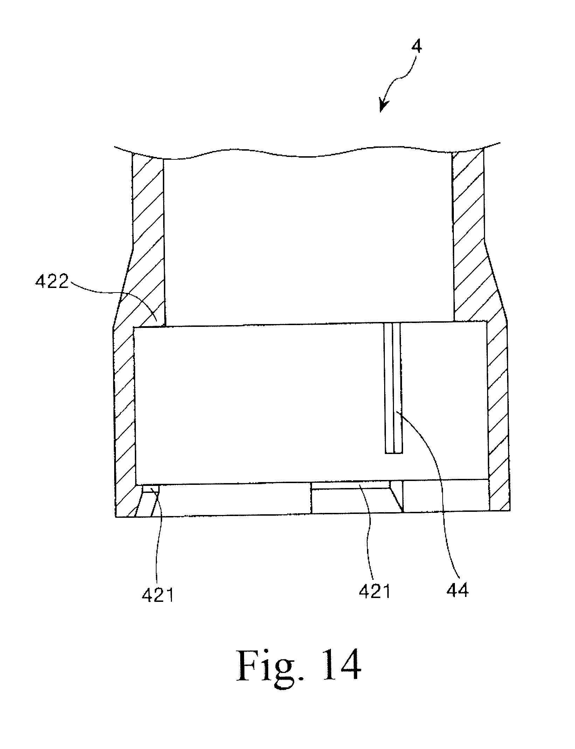

   Fig. 14 is a sectional view showing a proximal portion of an outer tube of the first connector of the connector assembly shown in Fig. 1.
[Fig. 15]

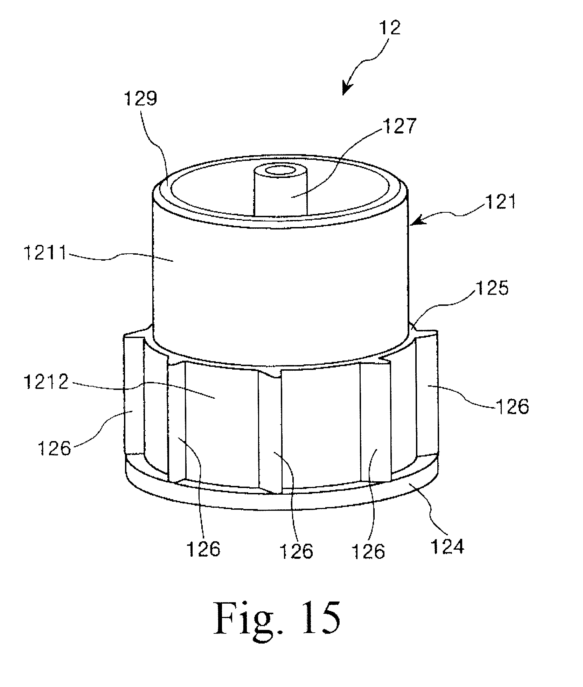

   Fig. 15 is a perspective view showing a hub of the first connector of the connector assembly shown in Fig. 1.
[Fig. 16]

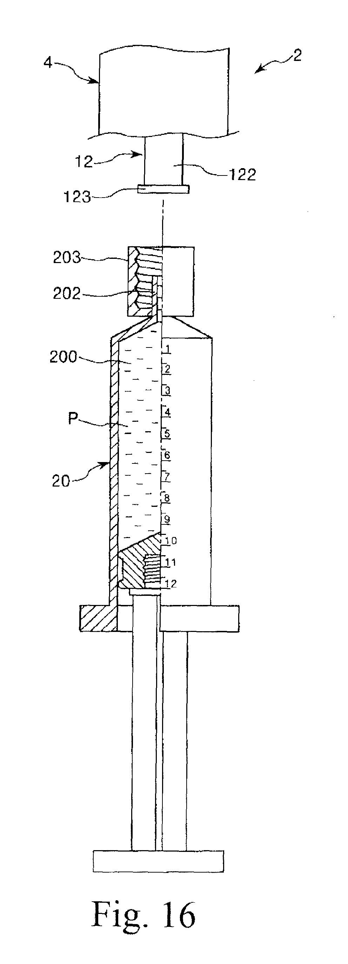

   Fig. 16 is a partial vertical sectional view showing a syringe which is connected to the first connector of the connector assembly shown in Fig. 1.
[Fig. 17]

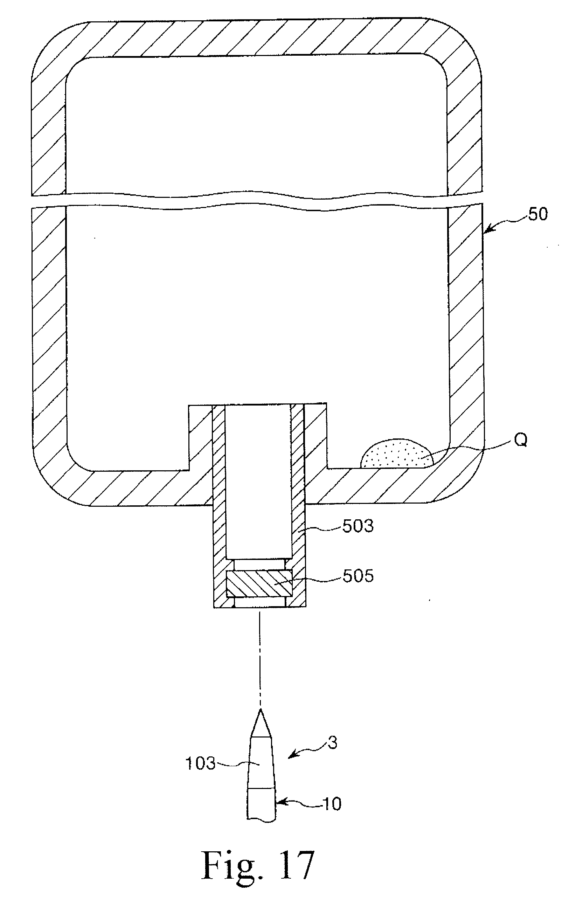

   Fig. 17 is a vertical sectional view of a bag connected to the second connector of the connector assembly shown in Fig. 1.

### Mode for Carrying Out the Invention

In the following, a connector and a connector assembly of the present invention are described in detail based on a preferred embodiment shown in the accompanying drawings.

Fig. 1 is an exploded perspective view showing an embodiment of a connector assembly of the present invention; Figs. 2 to 5 are vertical sectional views illustrating a process until a first connector and a second connector of the connector assembly shown in Fig. 1 are placed into an assembled state; Figs. 6 to 9 are perspective views illustrating a process until the first connector and the second connector of the connector assembly shown in Fig. 1 are placed into the assembled state (views corresponding to Figs. 2 to 5, respectively); Fig. 10 is a sectional view taken along line A-A of Fig. 3; Fig. 11 is a sectional view taken along line B-B of Fig. 4; Fig. 12 is a perspective view showing elements in the proximity of a proximal end portion of the first connector of the connector assembly shown in Fig. 2; Fig. 13 is a sectional perspective view taken along line C-C of Fig. 12; Fig. 14 is a vertical sectional view showing a proximal portion of an outer tube of the first connector of the connector assembly shown in Fig. 1; Fig. 15 is a perspective view showing a hub of the first connector of the connector assembly shown in Fig. 1; Fig. 16 is a partial vertical sectional view showing a syringe which is connected to the first connector of the connector assembly shown in Fig. 1; and Fig. 17 is a vertical sectional view of a bag connected to the second connector of the connector assembly shown in Fig. 1. In Fig. 16, for the convenience of description, a part of the outer tube of the first connector and a part of the hub of the first connector are omitted.

It is to be noted that, in the following description, for the convenience of description, an upper side in each of Figs. 1 to 11, and 14 to 17 is referred to as "distal end", "upper" or "upward", and a lower side as "proximal end", "lower" or "downward".

As shown in Figs. 1 to 17, a connector assembly 1 has a first connector (female connector) 2 and a second connector (male connector) 3. As shown in Fig. 16, the first connector 2 is mounted on a syringe (first medical device) 20. As shown in Fig. 17, the second connector 3 is mounted on a bag (second medical device) 50. This connector assembly 1 is used, in an assembled state (state illustrated in Figs. 5 and 9) in which the first connector 2 and the second connector 3 are assembled to each other by insertion of the second connector 3 into the first connector 2 from a distal end side of the first connector 2, to feed liquid from a first connector 2 side to a second connector 3 side or in an opposite direction.

As shown in Fig. 17, the bag 50 accommodates drug Q in a form of powder. A mouth portion 503 configured from a hard pipe is provided at a proximal end portion of the bag 50. Liquid can enter and leave the bag 50 through the mouth portion 503.

Further, a rubber stopper 505 for sealing the mouth portion 503 is mounted at the mouth portion 503. The rubber stopper 505 is punctured by a bottle needle portion 103 of the second connector 3. In this punctured state, the second connector 3 and the bag 50 communicate with each other.

Although the drug Q to be accommodated in the bag 50 is not limited specifically, for example, a drug which is dangerous if a health care worker touches by mistake such as an anti-cancer agent or an immunosuppressive agent, a drug which requires dissolution when it is to be used such as an antibiotic or a hemostatic agent, a drug which requires dilution such as a drug for pediatric use, a drug which is to be dealt out by a plural number of times such as vaccine, heparin and a drug for pediatric use and so forth are available. Further, the drug Q is not limited to drug in the form of powder but may be drug in a form of liquid.

Further, as shown in Fig. 16, a lock adapter 203 is provided on an outer circumferential portion of a mouth portion 202 provided at a distal end portion of the syringe 20. The lock adapter 203 has, on an inner circumferential face thereof, screw threads serving as a screw threaded portion for being screwed with a protrusion 123 of a hub portion 122. In the present embodiment, the lock adapter 203 is fixed to the mouth portion 202.

When the syringe 20 is to be connected to the hub portion 122 of a hub 12 of the first connector 2, the mouth portion 202 of the syringe 20 is fitted with the proximal end portion of the hub portion 122 of the hub 12 hereinafter described of the first connector 2, and the lock adapter 203 is rotated together with the syringe 20 so that the screw threads formed on the inner circumferential face of the lock adapter 203 are screwed with the protrusion 123 formed on the hub portion 122. In the following description, the screwing engagement between the protrusion 123 and the screw threads of the lock adapter 203 is merely referred to also as "screwing engagement between the protrusion 123 and the lock adapter 203". By the screwing engagement, the hub portion 122 of the hub 12 and the mouth portion 202 of the syringe 20 are connected to each other and the protrusion 123 and the lock adapter 203 are screwed with each other, and consequently, the syringe 20 is held by the hub 12. It is to be noted that, in this state, the hub portion 122 is accommodated in the lock adapter 203.

It is to be noted that, while the lock adapter 203 in the present embodiment is fixed to the mouth portion 202, the lock adapter 203 is not limited to this, but, for example, the lock adapter 203 may be disposed for movement along an axial direction of the syringe 20 with respect to the mouth portion 202, for rotation around the axis (coaxially), for movement along the axial direction and also for rotation around the axis, or the like.

Now, the connector assembly 1 is described. As described hereinabove, the connector assembly 1 has the first connector 2 and the second connector 3.

As shown in Figs. 2 to 5 and 12 to 15, the first connector 2 includes a first connector main body configured from an outer tube 4 of a cylindrical shape, an inner tube 7 of a cylindrical shape, and a hub 12 disposed at a proximal end portion of the outer tube 4. The first connector 2 further includes a hollow needle 5 supported on the hub 12, a first sealing member 6 supported on the inner tube 7, a coil spring 8 serving as puncturing portion biasing means for biasing the first sealing member 6 in a direction toward the distal end thereof, and a clamping member 9 disposed on the outer tube 4.

As shown in Figs. 1, 2, and 12 to 14, the outer tube 4 has the cylindrical shape. The second connector 3 is inserted into the outer tube 4 from an opening at the distal end of the outer tube 4, and the first connector 2 and the second connector 3 are connected to each other.

A plurality of (four in the configuration shown) protrusions 421 which project toward the inner side (inner side in a radial direction) are formed on an inner circumferential portion at a proximal end portion of the outer tube 4. The protrusions 421 are disposed at equal angular intervals along a circumferential direction.

Further, a stepped portion 422 is formed on the distal end side relative to the protrusions 421 of the inner circumferential portion of the outer tube 4.
Further, a pawl (first engaging portion) 44 which projects to the inner side of the outer tube 4 is formed between the protrusions 421 and the stepped portion 422 of the inner circumferential portion or the outer tube 4. Further, the pawl 44 has an elongated shape and extends along a axial direction. This pawl 44 cooperates with ratchet teeth 126 hereinafter described of the hub 12 to configure a ratchet mechanism. It is to be noted that a number of such pawls 44 is not limited to one but may be a plural number.

As shown in Figs. 1, 2, 12, 13 and 15, the hub 12 is provided at the proximal end portion of the outer tube 4 for rotation around the axis of the outer tube 4 with respect to the outer tube 4.

The hub 12 includes a main body portion 121 in a form of a tube and a hub portion (first connecting portion) 122 in a form of a tube projecting from a central portion of a partition 128 hereinafter described of the main body portion 121 in a direction toward the proximal end. The main body portion 121 and the hub portion 122 are disposed concentrically, and the hub portion 122 is positioned in the main body portion 121. Further, the main body portion 121 and the hub portion 122 of the hub 12, namely, the entire hub 12, is inserted in the outer tube 4. Consequently, an action to block a rotating operation of the hub 12 by a finger of a hand can be prevented.

The main body portion 121 has a small diameter portion 1211 and a large diameter portion 1212 which is positioned at the proximal end side of the small diameter portion 1211. Consequently, a stepped portion 125 is formed on a boundary between the small diameter portion 1211 and the large diameter portion 1212 on an outer circumferential portion of the main body portion 121. Further, a partition 128 for partitioning the inside of the main body portion 121 to the proximal end side and the distal end side is provided on the inner side of the small diameter portion 1211 in the proximity of the boundary between the small diameter portion 1211 and the large diameter portion 1212. The partition 128 is formed, in the configuration shown, as a plate.

A protrusion (projecting portion) 123 in a form of a flange is formed on an outer circumferential portion of the proximal end of the hub portion 122. The shape of the protrusion 123 is non-circular as viewed in an axial direction of the hub portion 122 and is such a shape that it is longer in one of two directions perpendicular to each other than in the other direction. The protrusion 123 is a portion for screwing with screw threads formed on the lock adapter 203 of the syringe 20. It is to be noted that the protrusion 123 is not limited to the protrusion 123 but may be, for example, screw threads or the like only if it can be screwed with the screw threads formed on the lock adapter 203.

At a central portion of the partition 128 of the main body portion 121, a post 127 of a tubular shape is formed such that it projects in a direction toward the distal end. The main body portion 121 and the post 127 are disposed concentrically.

The hollow needle 5 is inserted in the post 127 and fixed at the proximal end portion thereof to the partition 128. In other words, the hollow needle 5 is supported on the hub 12. Further, the partition 128 has an opening formed at a position thereof corresponding to a lumen (first flow path 52) of the hollow needle 5, and the hub portion 122 and the hollow needle 5 are communicated with each other through the opening.

Further, as described hereinabove, by screwing the protrusion 123 of the hub portion 122 and the lock adapter 203 of the syringe 20 with each other, the first connector 2 is mounted on the syringe 20, and the first connector 2 can be used in this mounted state (refer to Fig. 16). Further, in the mounted state, a space 200 of the syringe 20 and the lumen (first flow path 52) of the hollow needle 5 are communicated with each other through the hub portion 122. Liquid P for dissolving can be supplied from the syringe 20 into the hollow needle 5 through the hub portion 122.

Further, a flange (protrusion) 124 is formed at an outer circumferential portion of the proximal end of the main body portion 121.
The flange 124 is positioned on the distal end side with respect to the protrusions 421 of the outer tube 4 and contacts with the protrusions 421. Further, the stepped portion 125 is positioned on the proximal end side of the stepped portion 422 of the outer tube 4 and contacts with the stepped portion 422. Consequently, the hub 12 can rotate (turn) around the axis of the outer tube 4, but cannot move in the axial direction of the outer tube 4. Accordingly, separation preventing means for preventing separation of the hub 12 from the outer tube 4 is configured from the flange 124 and stepped portion 125 of the hub 12 and the protrusions 421 and stepped portion 422 of the outer tube 4.

Further, a plurality of (eight in the configuration shown) ratchet teeth (second engaging portions) 126 engageable with the pawl 44 of the outer tube 4 and projecting toward the outer side (outer side in a radial direction) are formed on an outer circumferential portion of the large diameter portion 1212 of the main body portion 121. The ratchet teeth 126 are disposed at equal angular intervals along the circumferential direction. Further, the ratchet teeth 126 has an elongated shape and extend along the axial direction.

The ratchet teeth 126 and the pawl 44 of the outer tube 4 configure rotation blocking means having a rotation blocking function for blocking the rotation of the hub 12 in only one of a forward direction and a backward direction.

In this instance, the "one" is a direction in which, when the syringe 20 is rotated with respect to the hub 12 to screw the lock adapter 203 of the syringe 20 with the protrusion 123 of the hub 12, the hub 12 is urged to rotate by rotational torque (rotational force) applied to the hub 12. In particular, a direction in which the hub 12 is urged to rotate by the rotational torque applied to the hub 12 and a direction of the rotation of the hub 12 blocked by the rotation blocking means coincide with each other. In other words, while deformation of the ratchet teeth 126 and the pawl 44 is permitted in the direction in which the rotation is permitted, in the reverse direction, passing of them each other by such deformation is disabled.

It is to be noted that, with the configuration shown in the figures, the rotation of the hub 12 in the clockwise direction in FIG. 13 with respect to the outer tube 4 is blocked while rotation in the counterclockwise direction is permitted. Further, the clockwise direction in FIG. 13 is a direction in which the hub 12 is urged to rotate by the rotational torque applied to the hub 12 when the syringe 20 is rotated with respect to the hub 12 to screw the lock adapter 203 of the syringe 20 with the protrusion 123 of the hub 12. Meanwhile, the counterclockwise direction in FIG. 13 is an opposite direction to that described above, namely, a direction in which the hub 12 is urged to rotate by the rotational torque applied to the hub 12 when the screwing engagement between the protrusion 123 and the lock adapter 203 is loosened.

As described hereinabove, when the pawl 44 of the outer tube 4 and the ratchet teeth 126 of the hub 12 are engaged with each other, the rotation blocking function of the rotation blocking means is rendered effective so that the rotation of the hub 12 in only one of the forward direction and the reverse direction with respect to the outer tube 4, namely, the rotation only in the clockwise direction in FIG. 13, is blocked. Consequently, the lock adapter 203 of the syringe 20 can be screwed with the protrusion 123 of the hub 12 and the syringe 20 can be mounted on the hub 12.

On the other hand, even if the syringe 20 is rotated with the intention of trying to remove the syringe 20 from the hub 12, the hub 12 rotates with respect to the outer tube 4 and the screwing engagement between the protrusion 123 and the lock adapter 203 cannot be loosened and the syringe 20 cannot be removed. Consequently, particularly a drug which is dangerous if a health care worker touches by mistake such as an anti-cancer agent or an immunosuppressive agent can be prevented from sticking to the health care worker.

Further, the hub 12 can rotate in one direction with respect to the outer tube 4, and consequently, by rotating the syringe 20 together with the hub 12 in a state in which the syringe 20 is mounted on the hub portion 122, graduations provided on the syringe 20 can be watched readily.

Further, as shown in Figs. 6 to 9, a grooved portion 48 is formed intermediately of the wall portion of the outer tube 4 such that it extends through the wall portion. This grooved portion 48 has an "L" shape as viewed in side elevation and is configured from a horizontal groove 481 formed along a circumferential direction of the wall portion of the outer tube 4, and a vertical groove 482 formed in a direction toward the proximal end along the axial direction of the outer tube 4 from one end of the horizontal groove 481. A protrusion 76 of the inner tube 7 is inserted in the grooved portion 48. The protrusion 76 of the inner tube 7 can move in the grooved portion 48.

As shown in Figs. 2 to 5, a pair of grooved portions 43 is formed at a distal end portion of the wall portion of the outer tube 4, wherein the grooved portions are formed in an opposing relationship to each other through the center axis of the outer tube 4. Two clamping members 9 each having a ring shape are inserted in a superposed state in the grooved portions 43. The clamping members 9 function as part of a stopper 17 for restricting the movement of the second connector 3 (second connector main body 10) in a direction toward the distal end thereof in the outer tube 4. As the configuration of the stopper 17, a publicly known configuration (for example, the configuration of the "hub attaching and detaching mechanism" disclosed in Japanese Patent Laid-Open No. H08-126630) can be used.

In this instance, each clamping member 9 has, at a portion of an outer circumferential portion thereof, an operation portion 92 operable for pressing the clamping member 9. By pressing the operation portions 92, the clamping members 9 move in a direction perpendicular to the axis of the outer tube 4.

Further, each of the clamping members 9 has a plurality of inwardly projecting protrusions (first engaging portions) 91 at portions thereof on the opposite side to the operation portion 92. The protrusions 91 of one of the clamping members 9 and the protrusions 91 of the other clamping member 9 are disposed in an opposing relationship to each other through the center axis of the outer tube 4.

Further, each of the clamping members 9 has a pair of elastic pieces 93 projecting from an outer circumferential portion thereof on the same side as the protrusions 91. The elastic pieces 93 of one of the clamping members 9 make contact with the inner side of the operation portion 92 of the other clamping member 9. Similarly, the elastic pieces 93 of the other clamping member 9 make contact with the inner side of the operation portion 92 of the one clamping member 9.

When a pressing operation of the clamping members 9 is carried out, the pressing operation is carried out against the biasing force (elastic force) of the elastic pieces 93. By this operation, the protrusions 91 of one of the clamping members 9 and the protrusions 91 of the other clamping member 9 are placed into a mutually spaced state. Then, if the pressing force to the clamping members 9 is canceled, then the protrusions 91 of the one clamping member 9 and the protrusions 91 of the other clamping member 9 are placed into a mutually closely positioned state by the biasing force of the elastic pieces 93.

In the state in which the protrusions 91 of the one clamping member 9 and the protrusions 91 of the other clamping member 9 are closely positioned, the protrusions 91 collectively engage with an engaging portion (second engaging portion) 105a or 105b of the second connector 3 (see Figs. 3 to 5). Consequently, inadvertent separation of the second connector 3 from the outer tube 4 can be prevented with certainty.

Further, in the state in which the protrusions 91 of the one clamping member 9 and the protrusions 91 of the other clamping member 9 are spaced from each other, the engagement of the clamping members 9 and the second connector 3 is canceled.

As shown in Fig. 1, the engaging portions 105a and 105b of the second connector 3 are each configured from a flange portion formed on an outer circumferential portion of the second connector main body 10 and having an increased diameter. The engaging portions 105a and 105b are disposed in a spaced relationship from each other along the axial direction of the second connector main body 10. Thus, as shown in Figs. 3 and 5, one of the engaging portions 105a and 105b is engaged with the protrusions 91 as described above in response to the insertion depth of the second connector 3 in the first connector.

In the connector assembly 1, the clamping members 9 and the engaging portions 105a and 105b of the second connector 3 configure the "stopper 17" for locking the outer tube 4 and the second connector 3.

As shown in Figs. 2 and 6, a plurality of (four in the present embodiment) inwardly projecting stepped portions 49 are formed at a portion of the wall portion of the outer tube 4 between the grooved portion 48 and the grooved portions 43 of an inner circumferential portion 47 of the outer tube 4. As shown in Fig. 2, as the inner tube 7 makes contact with each stepped portion 49, the movement of the inner tube 7 in a direction toward the distal end thereof can be restricted, and consequently, the inner tube 7 can be prevented from separating from the outer tube 4 with certainty.

As shown in Fig. 2 (similarly in Figs. 3 to 5), the inner tube 7 is disposed on the inner side of the outer tube 4. This inner tube 7 is displaceable with respect to the outer tube 4, in particular, rotatable around the axis of the outer tube 4 and movable along the axial direction of the outer tube 4.

The inner tube 7 has a sealing member disposition portion 73 on which the first sealing member 6 is placed. The sealing member disposition portion 73 is provided on the inner side of the inner tube 7 and configured from a pair of annular plate-shaped portions 731 and 732 which sandwich the first sealing member 6 from above and below.

Further, the inner tube 7 has a sliding member 74 which slidably moves on the hollow needle 5 when the inner tube 7 is displaced, and a fixing portion 75 for fixing the sliding member 74. The sliding member 74 is a member having a tubular shape and configured from an elastic material having a reduced diameter portion 741 of a reduced diameter. Although the configuring material of the sliding member 74 is not specifically limited, for example, various rubber materials of natural rubber, butyl rubber, isoprene rubber, butadiene rubber, styrenebutadiene rubber, silicone rubber and so forth, various thermoplastic elastomers of polyurethane-based, polyester-based, polyamide-based, olefin-based and styrene-based types and the like, or elastic materials of mixtures of them and so forth can be used. When the inner tube 7 is displaced, the reduced diameter portion 741 makes contact with and slidably moves on an outer circumferential portion 54 of the hollow needle 5. The fixing portion 75 is a portion of a tubular shape formed integrally with the plate-shaped portion 732 so as to project downwardly from a plate-shaped portion 732.

As shown in Fig. 6 (similarly in Figs. 7 to 9), the protrusion 76 is formed in a projecting manner on an outer circumferential portion of the wall portion of the inner tube 7. This protrusion 76 is inserted in the grooved portion 48 of the outer tube 4 and moves in the grooved portion 48 in response to displacement of the inner tube 7. Consequently, the first connector 2 can assume a first state (state illustrated in Figs. 6 and 7) in which the protrusion 76 is positioned in the horizontal groove 481, a second state (state illustrated in Fig. 8) in which the protrusion 76 is positioned at a crossing portion 483 between the horizontal groove 481 and the vertical groove 482 as a result of rotational operation of the inner tube 7 with respect to the outer tube 4 from the first state, and a third state (state illustrated in Fig. 9) in which the protrusion 76 is positioned in the vertical groove 482 as a result of a pushing-in operation of the inner tube 7 with respect to the outer tube 4 from the second state.

If the second connector 3 is inserted into the first connector 2 in the first state illustrated in Fig. 6 (Fig. 2) (this operation is hereinafter referred to as "insertion operation"), then a second sealing member 11 of the second connector 3 makes contact with the first sealing member 6 of the first connector 2 and presses the first sealing member 6 in a direction toward the proximal end thereof thereby to tend to move the first sealing member 6 together with the inner tube 7. However, since the protrusion 76 of the inner tube 7 is positioned in the horizontal groove 481 of the outer tube 4, the movement of the inner tube 7 in the direction toward the proximal end is restricted (refer to Figs. 3 and 7).

If, from the state illustrated in Fig. 7, for example, a finger is put to the protrusion 76 of the inner tube 7 to operate the inner tube 7 to rotate ina direction indicated by an arrow mark as seen in Fig. 8, then the first connector 2 is in the second state as shown in Fig. 8. Then, the restriction of the movement of the inner tube 7 in the direction toward the proximal end is canceled thereby to allow the movement of the inner tube 7. Consequently, the insertion operation can be re-started. It is to be noted that, also in the second state, the closely contacting state of the first sealing member 6 and the second sealing member 11 is maintained as seen in Fig. 4. Further, in the second state, the inner tube 7 and the second connector 3 (second connector main body 10) are fixed by the locking means 19. Consequently, the closely contacting state between a first puncture portion 61 of the first sealing member 6 and a second puncture portion 111 of the second sealing member 11 is fixed. This locking means 19 is hereinafter described.

If the second connector 3 is pushed in the direction toward the proximal end from the state illustrated in Fig. 8 against the biasing force of the coil spring 8, then the insertion operation is re-started, and the first connector 2 is placed into the third state illustrated in Fig. 9. It is to be noted that, also in the third state, the closely contacting state between the first sealing member 6 and the second sealing member 11 is maintained as seen in Fig. 5.

If the second connector 3 is pulled out from the first connector 2 in the state (assembled state) illustrated in Fig. 9 (this operation is hereinafter referred to as "pulling out operation"), then the inner tube 7 is moved in the direction toward the distal end together with the second connector 3 by the biasing force of the coil spring 8 reversely to that described hereinabove. Consequently, the first connector 2 is placed into the second state illustrated in Fig. 8. In this second state, further movement in the direction toward the distal end of the protrusion 76 of the inner tube 7 is restricted. Consequently, the pulling out operation can be restricted halfway once.

Further, if the inner tube 7 is operated to rotate in the opposite direction to that described above from the second state, then the first connector 2 is placed into the first state illustrated in Fig. 7. Consequently, the locked state between the inner tube 7 and the second connector 3 by the locking means 19 is canceled thereby to allow movement only of the second connector 3 in the direction toward the distal end. Consequently, the pulling out operation can be re-started. If the pulling out operation is re-started, then the first connector 2 and the second connector 3 are placed back into the disassembled state illustrated in Fig. 6.

In this manner, in the connector assembly 1, restriction of an insertion operation, cancellation of the restriction of the insertion operation, restriction of a pulling out operation and cancellation of the restriction of the pulling out operation are carried out in response to the position of the protrusion 76 of the inner tube 7 with respect to the grooved portion 48 of the outer tube 4. Accordingly, the protrusion 76 of the inner tube 7 and the grooved portion 48 of the outer tube 4 configure "operation restriction means 18" for restricting such operations as described above.

As shown in Fig. 2, a plurality of (four in the present embodiment) engaging pieces (elastic pieces) 77 are formed at a distal end portion of the inner tube 7 such that they project in the direction toward the distal end. Each of the engaging pieces 77 has a pawl 771 provided at a distal end portion thereof and engageable with a recessed portion (engaging portion) 101a of the second connector 3.

It is to be noted that the recessed portion 101a is a portion formed in a ring shape along a circumferential direction at a distal end portion of an outer circumferential portion 101 of the second connector main body 10.

In a state in which the second connector 3 is not inserted in the first connector 2 as yet, each of the engaging pieces 77 is inclined outwardly. Consequently, in a state in which the second connector 3 is inserted in the first connector 2, each engaging piece 77 can assume a state (state illustrated in Figs. 3 and 10) in which it is spaced away from the recessed portion 101a of the second connector 3 and another state (state illustrated in Figs. 4, 5 and 11) in which it engages with the recessed portion 101a as a result of movement toward the recessed portion 101a by being pushed by a pressing portion 471 of the outer tube 4. By this engagement, the inner tube 7 and the second connector 3 are locked with certainty.

It is to be noted that the pressing portion 471 is configured from a plurality of (four in the present embodiment) ribs formed along the axial direction of the inner circumferential portion 47 of the outer tube 4.

The pressing portions 471 are disposed at equal intervals along a circumferential direction of the outer tube 4. In the state illustrated in Fig. 10, one engaging piece 77 is positioned between each adjacent ones of the pressing portions 471, and pushing of the engaging piece 77 by the pressing portion 471 is not carried out as yet. At this time, the first connector 2 assumes the first state.

The engaging pieces 77 are disposed at equal intervals around the axis of the inner tube 7. By operating the inner tube 7 to rotate from the state illustrated in Fig. 10 as described above, the engaging pieces 77 climb inclined faces 472 of the pressing portions 471. As a result, one pressing portion 471 presses one engaging piece 77 against the elastic force of the engaging piece 77. Consequently, the four engaging pieces 77 engage uniformly with the second connector 3 along the circumferential direction thereof thereby to lock the inner tube 7 and the second connector 3 with a higher degree of certainty. At this time, the first connector 2 is in the second state. The state in which the pressing portion 471 presses the engaging piece 77 is maintained even if the first connector 2 is placed into the third state.

In this manner, in the connector assembly 1, the engaging pieces 77 of the inner tube 7, the pressing portions 471 of the outer tube 4 and the recessed portion 101a of the second connector 3 configure "locking means 19" for locking the inner tube 7 and the second connector 3 with certainty. The locking means 19 operates when the first connector 2 is placed from the first state into the second state. In other words, the locking means 19 operates in an interlocking relationship with a canceling operation of canceling the restriction of the insertion operation. On the contrary, the locking means 19 operates also when the first connector 2 is placed from the second state into the first state. In particular, the locking means 19 operates in an interlocking relationship also with a canceling operation for canceling the restriction of the pulling out operation. Consequently, while the first connector 2 is relatively displaced between the first state and the second state, in other words, before and after the hollow needle 5 punctures the first sealing member 6 and the second sealing member 11, the first sealing member 6 and the second sealing member 11 can be closely contacted with each other with certainty and besides, upon pulling out, inadvertent pulling out (coming out) only of the second connector 3 can be prevented.

It is to be noted that, although the configuring material of the outer tube 4, inner tube 7, clamping members 9, and hub 12 is not limited specifically, various resins such as, for example, polyvinyl chloride, polyethylene, polypropylene, a cyclic polyolefin, polystyrene, poly-(4-methyl pentene-1), polycarbonate, acrylic resins, an acrylonitrile-butadiene-styrene copolymer, a polyester such as polyethylene terephthalate or polyethylene naphthalate, a butadiene-styrene copolymer, and a polyamide (for example, nylon 6, nylon 6.6, nylon 6.10 or nylon 12) are applicable. It is preferable to use, among them, such resins as polypropylene, the cyclic polyolefin and the polyester in that they are easy to mold and low in water vapor permeability.

As shown in Fig. 2, the hollow needle 5 configured from a metal material is disposed on the axis of the outer tube 4. As described hereinabove, the hollow needle 5 is supported at a proximal end portion thereof on the hub 12.

The hollow needle 5 has a form of a tube and has a lumen which functions as the first flow path 52 along which liquid P for dissolving (liquid) can pass. Further, the hollow needle 5 is closed at the distal end thereof and has a side hole (opening) 53 formed such that it is open to a distal end portion of a wall portion thereof. The side hole 53 is communicated with the first flow path 52.

A sharp needle tip 51 is formed at the distal end of the hollow needle 5. As shown in Fig. 5, the needle tip 51 can puncture the first sealing member 6 of the first connector 2 and the second sealing member 11 of the second connector 3 hereinafter described. As shown in Fig. 5, in an assembled state, the hollow needle 5 is exposed, in a region thereof from the needle tip 51 to the portion at which the side hole 53 is formed, to the lumen of the second connector 3. Consequently, the lumen of the hollow needle 5 and the lumen of the second connector 3 are communicated with each other through the side hole 53 of the hollow needle 5. In other words, the first flow path 52 of the first connector 2 and a second flow path 102 of the second connector 3 hereinafter described are communicated with each other.

As shown in Fig. 2, the first sealing member 6 is disposed on the inner side of the inner tube 7. This first sealing member 6 seals a lumen portion of the inner tube 7 and has a form of a disk. The first sealing member 6 is disposed such that the thicknesswise direction thereof coincides with the axial direction of the inner tube 7. Consequently, when the first sealing member 6 moves toward the proximal end side along the axial direction of the hollow needle 5, the first sealing member 6 is punctured readily and with certainty by the needle tip 51 of the hollow needle 5.

The first sealing member 6 is an elastic member having a thickness at a central portion thereof greater than that at an edge portion thereof. This central portion serves as a first puncture portion 61 which is punctured by the hollow needle 5. Meanwhile, the first sealing member 6 is sandwiched at an edge portion thereof by the pair of plate-shaped portions 731 and 732 of the inner tube 7 as described hereinabove. Consequently, the first sealing member 6 is fixed with certainty to the inner tube 7 and can move together with the inner tube 7.

Further, the connector assembly 1 is set such that a total of sliding resistance between the portions of the first sealing member 6 (first puncture portion 61) and the second sealing member 11 (second puncture portion 111) which are punctured by the hollow needle 5 and the outer circumferential portion 54 of the hollow needle 5 contacting with the portions, and sliding resistance between the reduced diameter portion 741 of the sliding member 74 and the outer circumferential portion 54 of the hollow needle 5 contacting with the reduced diameter portion 741 is smaller than the biasing force of the coil spring 8. Consequently, when the first connector 2 cancels the stopper 17 which is in the third state illustrated in Fig. 5, the first connector 2 can return to the second state illustrated in Fig. 4 by the biasing force of the coil spring 8. It is to be noted that, although a setting method of the relationship in magnitude between the forces is not limited specifically, such methods as selection of the configuration material of the first sealing member 6, second sealing member 11 or sliding member 74, adjustment of the thickness of the first puncture portion 61 or the second puncture portion 111, selection of the configuration material of the coil spring 8, adjustment of the wire diameter or the number of windings of the coil spring 8, adjustment of the outer diameter of the hollow needle 5 and so forth are available.

As shown in Fig. 2, the first puncture portion 61 has a distal end face 612 which is elevated in a natural state thereof in which no external force is applied. Then, in a closely contacting state in which the first sealing member 6 and the second sealing member 11 closely contact with each other as seen in Fig. 3, the distal end face 612 which has been elevated is crushed. Consequently, the closely contacting state becomes more reliable, and therefore, the liquid-tightness on a boundary between the first sealing member 6 and the second sealing member 11 can be assured. Consequently, feeding of the liquid in the assembled state can be carried out safely and with certainty.

It is to be noted that the configuring material of the first sealing member 6 is not limited specifically, and similar materials to those listed as the configuring material of the sliding member 74 described hereinabove can be used.

As shown in Fig. 2, the coil spring 8 configured from a metal material such as stainless steel is installed in the outer tube 4. The coil spring 8 is in a compressed state and is in contact at the distal end thereof with the plate-shaped portion 732 of the inner tube 7 and at the proximal end thereof with the partition 128 (bottom portion) of the hub 12. Consequently, the first sealing member 6 can be biased with certainty in the direction toward the distal end through the inner tube 7. The hollow needle 5 is fitted in the coil spring 8. In other words, the coil spring 8 is disposed around the hollow needle 5 along the hollow needle 5. It is to be noted that the biasing means may be formed, in addition to the coil spring 8, from a bellows-like leaf spring or a cylindrical or bellows-like rubber member.

As shown in Figs. 1 and 2, the second connector 3 has the second connector main body 10 of a tubular shape, and the second sealing member 11 provided on the second connector main body 10.

The second connector main body 10 is a member having a cylindrical shape. The lumen of the second connector main body 10 functions as the second flow path 102 along which liquid can pass. As shown in Fig. 17, a distal end portion (second connection portion) of the second connector main body 10 serves as the bottle needle portion 103 having a tapering shape and can puncture the rubber stopper 505 of the bag 50. Further, a side hole (not shown) is formed in the bottle needle portion 103. If the bottle needle portion 103 punctures the rubber stopper 505 of the bag 50 until the side hole is exposed in the bag 50, then the inside of the bag 50 and the second flow path 102 are communicated with each other. Consequently, liquid passing the second flow path 102 can be supplied into the bag 50.

Further, as described hereinabove, the engaging portions 105a and 105b which engage with the protrusions 91 of the first connector 2 are formed intermediately of the second connector main body 10.

Further, a plurality of (four in the present embodiment) ribs 104 are formed along a longitudinal direction on an outer circumferential portion of the second connector main body 10. The ribs 104 are disposed at equal intervals along a circumferential direction of the outer circumferential portion of the second connector main body 10. The second connector main body 10 can be reinforced by the ribs 104.

The second connector main body 10 has, at a proximal end portion thereof, a sealing member installation portion 106 at which the second sealing member 11 is disposed. The sealing member installation portion 106 is configured from a pair of ring-shaped plate-like portions 106a and 106b which sandwich the second sealing member 11 from above and below.

It is to be noted that, although the configuration material of the second connector main body 10 is not limited specifically, for example, such materials as listed in the descriptions of the outer tube 4, inner tube 7, clamping member 9 and hub 12 of the first connector 2 can be used.

As shown in Fig. 2, the second sealing member 11 seals a lumen portion of the second connector main body 10 and has a form of a disk. The second sealing member 11 is disposed such that the thicknesswise direction thereof coincided with the axial direction of the second connector main body 10. Consequently, the second sealing member 11 can be punctured readily and with certainty by the needle tip 51 of the hollow needle 5 together with the first sealing member 6 closely contacting therewith.

Further, the second sealing member 11 is an elastic member having a thickness greater at a central portion thereof than that at an edge portion thereof. The central portion of the second sealing member 11 servers as the second puncture portion 111 which is punctured by the hollow needle 5. Meanwhile, the second sealing member 11 is sandwiched at the edge portion thereof by the pair of plate-like portions 106a and 106b of the second connector main body 10 as described hereinabove. Consequently, the second sealing member 11 is fixed with certainty to the second connector main body 10.

As shown in Fig. 2, when the second puncture portion 111 is in a natural state in which no external force is applied thereto, the proximal end face 112 thereof is elevated. In the closely contacting state in which the first sealing member 6 and the second sealing member 11 closely contact with each other as shown in Fig. 2, the proximal end face 112 which has been elevated is crushed similarly to the distal end face 612 of the first sealing member 6. Consequently, the closely contacting state becomes more sure. Therefore, the liquid-tightness at the boundary portion between the first sealing member 6 and the second sealing member 11 can be assured.

It is to be noted that the configuration material of the second sealing member 11 is not limited specifically, and for example, materials similar to those listed as the configuration materials of the sliding member 74 can be used.

Now, an operation state when the connector assembly 1 is used is described.

### [1] Process from a non-assembled state to an assembled state (refer to the drawings in the order of Fig. 2 (Fig. 6) → Fig. 3 (Fig. 7) → Fig. 4 (Fig. 8) → Fig. 5 (Fig. 9) after the first connector 2 is mounted on the syringe 20)

First, the syringe 20 is mounted on the first connector 2, and the second connector 3 is mounted on the bag 50.

When the syringe 20 is to be mounted on the first connector 2, the mouth portion 202 of the syringe 20 is inserted into the proximal end portion of the hub portion 122 of the hub 12 of the first connector 2, and the lock adapter 203 is rotated together with the syringe 20. At this time, the ratchet tooth 126 of the hub 12 and the pawl 44 of the outer tube 4 are engaged with each other to block the rotation of the hub 12 with respect to the outer tube 4, and the lock adapter 203 is screwed with the protrusion 123 of the hub portion 122. In other words, the hub portion 122 and the mouth portion 202 of the syringe 20 are connected to each other and the protrusion 123 and the lock adapter 203 are screwed with each other so that the syringe 20 is retained on the hub 12.

In this manner, the syringe 20 mounted on the hub 12 cannot be removed from the hub 12. In other words, even if the syringe 20 is rotated with the intention of trying to remove the syringe 20 from the hub 12, the hub 12 rotates with respect to the outer tube 4 and the screwing engagement between the protrusion 123 and the lock adapter 203 cannot be loosened. Consequently, particularly a drug which is dangerous if a health care worker touches by mistake such as an anti-cancer agent or an immunosuppressive agent can be prevented from sticking to the health care worker.

Further, since the rotation of the hub 12 in the one direction is permitted, by rotating the syringe 20 together with the hub 12 in the state in which the syringe 20 is mounted on the hub portion 122, graduations provided on the syringe 20 can be watched readily.

Further, when the syringe 20 is to be mounted on the first connector 2, only it is necessary to screw the protrusion 123 and the lock adapter 203 with each other and there is no necessity to carry out any other operation. Therefore, the connection of the syringe 20 can be carried out readily and rapidly.

Thereafter, the second connector 3 in a non-assembled state is moved from the proximal end thereof toward the distal end portion of the first connector 2 as seen in Fig. 2. In the non-assembled state, the first connector 2 assumes the first state (state in which the protrusion 76 of the inner tube 7 is positioned in the horizontal groove 481 of the grooved portion 48 of the outer tube 4) (refer to Fig. 6). The first sealing member 6 is positioned on the distal end side with respect to the hollow needle 5.

As shown in Figs. 3 and 7, as the second connector 3 is inserted into the first connector 2, the distal end face 612 of the first sealing member 6 of the first connector 2 and the proximal end face 112 of the second sealing member 11 of the second connector 3 are brought into contact with each other and elastically deformed so that they closely contact with each other. At this time, since the first connector 2 is in the first state as described hereinabove (refer to Fig. 7), the insertion operation of the second connector 3 into the first connector 2 is restricted once.

Further, the stopper 17 operates (the clamping member 9 of the first connector 2 is engaged with the engaging portion 105a of the second connector 3) so that the second connector 3 moves reversely in the direction toward the distal end as seen in Fig. 3 thereby to prevent the second connector 3 from separating from the first connector 2.

Then, if the inner tube 7 of the first connector 2 is operated to rotate in the direction indicated by an arrow mark in Fig. 8, then the first connector 2 is placed into the second state (state in which the protrusion 76 of the inner tube 7 is positioned at the crossing portion 483 of the grooved portion 48 of the outer tube 4) as seen in Fig. 8. Consequently, the restriction of the insertion operation is canceled and the insertion operation can be re-started as described hereinabove.

Further, the assembled state of the first connector 2 and the second connector 3 is maintained by the stopper 17 and the locking means 19. Thus, the second connector 3 can be prevented from being pulled out from the first connector 2, or in other words, the connector assembly 1 in the assembled state can be prevented from being disassembled inadvertently. Consequently, the liquid P for dissolving can be fed safely through the connector assembly 1.

Further, in the assembled state, the close contact between the first sealing member 6 of the first connector 2 and the second sealing member 11 of the second connector 3 is maintained (refer to Fig. 5). Consequently, the liquid-tightness (air-tightness) particularly in the proximity of a joining portion of the first flow path 52 and the second flow path 102 can be maintained with certainty, and the liquid P for dissolving which passes the flow paths can be prevented from leaking from the connector assembly 1 in the assembled state with certainty.

Further, in the state illustrated in Fig. 5, the proximal end 78 of the inner tube 7 makes contact with the distal end 129 of the main body portion 121 of the hub 12. Consequently, a insertion limit of the second connector 3 is restricted.

### [2] Process from an assembled state back into a non-assembled state (refer to the drawings in the order of Fig. 5 (Fig. 9) → Fig. 4 (Fig. 8) → Fig. 3 (Fig. 7) → Fig. 2 (Fig. 6))

From the state illustrated in Figs. 5 and 9, the clamping members 9 are operated to cancel the locked state of the first connector 2 and the second connector. Consequently, a pulling out operation of pulling out the second connector from the first connector 2 can be started.

As seen in Figs. 4 and 8, when the pulling out operation is started, the second connector 3 moves in the direction toward the distal end conversely to that described hereinabove. At this time, since the biasing force of the coil spring 8 acts upon the first sealing member 6 through the inner tube 7, the first sealing member 6 can follow up the movement of the second connector 3. Consequently, also when the pulling out operation is carried out, the closely contacting state between the first sealing member 6 and the second sealing member 11 is maintained.

Then, when the first connector 2 is placed into the second state, the pulling out operation is restricted once as described hereinabove (refer to Fig. 8). At this time, the side hole 53 of the hollow needle 5 is positioned on the proximal end side with respect to the second sealing member 11 (in the configuration shown, the first sealing member 6 which is on the proximal end side further with respect to the second sealing member 11). It is to be noted that the first sealing member 6 and the second sealing member 11 are closed, at portions thereof punctured by the hollow needle 5, by the self closing property.

Then, if the inner tube 7 is operated to rotate in the opposite direction to that described hereinabove, then the first connector 2 is placed into the first state illustrated in Fig. 7. At this time, the locked state of the inner tube 7 and the second connector 3 by the locking means 19 is canceled as seen in Fig. 3 thereby to allow movement only of the second connector 3 in the direction toward the distal end. Consequently, the pulling out operation of the second connector 3 can be re-started.

After the pulling out operation is re-started, the first puncture portion 61 and the second puncture portion 111 which have been in the closely contacting state are spaced away from each other and the connector assembly 1 which has been in the assembled state can be placed back into the disassembled state as seen in Figs. 2 and 6.

In this manner, with the connector assembly 1, when the second connector 3 is to be pulled out from the first connector 2, the first sealing member 6 and the second sealing member 11 can be prevented from being spaced away from each other before the hollow needle 5 is pulled out fully from the second sealing member 11. Consequently, even while the connector assembly 1 in the assembled state is being disassembled, the liquid-tightness of the first flow path 52 and the second flow path 102 is maintained. Therefore, the solution (liquid) in the flow paths can be prevented from leaking from the connector assembly 1 with certainty. Consequently, feeding of the solution can be carried out safely using the connector assembly 1.

While the connector and the connector assembly of the present invention has been described above with reference to the embodiment shown in the drawings, the present invention is not limited to this, but the elements which configure the connector and the connector assembly can be replaced with elements of arbitrary configurations which can exhibit similar functions. Further, an arbitrary configuration element may be additionally provided.

Further, although the rotation blocking means is configured from the ratchet teeth provided on the hub and the pawl provided on the outer tube, the rotation blocking means is not limited to this. For example, the rotation blocking means may be configured from a pawl provided on the hub and ratchet teeth provided on the outer tube.

Further, while the operation restriction means is configured from a grooved portion formed on a wall portion of an outer tube and a protrusion formed in a projecting manner on a wall portion of an inner tube and configured to be inserted into the grooved portion, the operation restriction means is not limited to this. For example, the operation restriction means may be configured from a grooved portion formed on a wall portion of an inner tube, and a protrusion formed in a projecting manner on a wall portion of an outer tube and configured to be inserted into the grooved portion.

Further, while the first puncture portion and the second puncture portion are elevated at end faces thereof, the configuration of the first and second puncture portions is not limited to this. For example, only one of the puncture portions may be elevated at an end face thereof.
Further, the present invention may not have the coil spring 8.

### Industrial Applicability

With the invention, since a connector has rotation blocking means having a rotation blocking function for blocking rotation of a hub in only one of a forward direction and a backward direction, when a medical device is to be connected to the connector, the medical device can be rotated with respect to the hub to screw a screw threaded portion of the medical device with a projecting portion of the hub.
Then, after the medical device is connected to the connector, even if the medical device is rotated in a direction in which the screwing engagement between the projecting portion and the screw threaded portion is loosened, the hub rotates with respect to the outer tube. Therefore, the screwing engagement between the projecting portion and the screw threaded portion cannot be cancelled. Consequently, the medical device can be prevented from being separated from the connector.
Further, the medical device is permitted to rotate with respect to the connector in the connected state to the connector. Therefore, for example, during working for dissolving drug into dissolution liquid, graduations provided on the medical device can be watched readily. Accordingly, the present invention has industrial applicability.

## Claims

1. A connector, comprising:
an outer tube;
a hub disposed at a proximal end portion of the outer tube for rotation around an axis of the outer tube with respect to the outer tube, wherein the hub includes a main body portion inserted in the outer tube, a connecting portion having a proximal end side to which a medical device having a screw threaded portion is to be connected, and a projecting portion for being screwed with the screw threaded portion;
separation preventing means for preventing separation of the hub from the outer tube; and
rotation blocking means having a rotation blocking function for blocking the rotation of the hub in only one of a forward direction and a backward direction;
wherein the rotation blocking means includes a first engaging portion provided at the proximal end portion of the outer tube and a second engaging portion provided on the hub and engageable with the first engaging portion,
wherein a direction in which the hub is urged to rotate by rotational torque applied to the hub when the medical device is rotated with respect to the hub to screw the screw threaded portion with the projecting portion and a direction of the rotation of the hub blocked by the rotation blocking means coincide with each other, and
wherein the rotation blocking function of the rotation blocking means is rendered effective when the first engaging portion and the second engaging portion are engaged with each other.

2. The connector according to claim 1, wherein one of the first engaging portion and the second engaging portion is a pawl and the other of the first engaging portion and the second engaging portion is a ratchet tooth engageable with the pawl.

3. The connector according to claim 2, wherein the ratchet tooth includes a plurality of ratchet teeth, and the plurality of ratchet teeth are provided along a circumferential direction.

4. The connector according to claim 1, wherein the medical device includes a syringe having a mouth portion at a distal end portion thereof; and
in the connector, the mouth portion of the syringe is connected to the connecting portion.

5. A connector assembly, comprising:
the connector according to claim 1; and
a partner connector connected to the connector and having a connecting portion, wherein the connecting portion connects, at a distal end side of the connecting portion, a liquid accommodating container capable of accommodating liquid.
